(19) Europäisches Patentamt
European Patent Office
Office européen des brevets

(11) EP 1 337 222 B1

(12) **EUROPÄISCHE PATENTSCHRIFT**

(45) Veröffentlichungstag und Bekanntmachung des Hinweises auf die Patenterteilung:
**26.03.2008 Patentblatt 2008/13**

(21) Anmeldenummer: **01997278.5**

(22) Anmeldetag: **19.11.2001**

(51) Int Cl.:
***A61K 6/083*** *(2006.01)*

(86) Internationale Anmeldenummer:
**PCT/EP2001/013350**

(87) Internationale Veröffentlichungsnummer:
**WO 2002/041845 (30.05.2002 Gazette 2002/22)**

(54) **VERWENDUNG VON POLYSÄUREN MIT ENGER MOLMASSENVERTEILUNG**

UTILIZATION OF POLYACIDS HAVING A TIGHT MOLAR MASS DISTRIBUTION

UTILISATION DE POLYACIDES AYANT UNE REPARTITION DE MASSE MOLAIRE RESTREINTE

(84) Benannte Vertragsstaaten:
**AT BE CH CY DE DK ES FI FR GB GR IE IT LI LU MC NL PT SE TR**

(30) Priorität: **27.11.2000 DE 10058829**

(43) Veröffentlichungstag der Anmeldung:
**27.08.2003 Patentblatt 2003/35**

(73) Patentinhaber: **3M ESPE AG**
**82229 Seefeld (DE)**

(72) Erfinder:
- **MIKULLA, Markus**
**82346 Andechs-Frieding (DE)**
- **RACKELMANN, Gabriele**
**82205 Gilching (DE)**
- **STEFAN, Klaus-Peter**
**82229 Seefeld (DE)**

(56) Entgegenhaltungen:
**EP-A- 0 024 056**
**DE-A- 2 439 882**
**DE-A- 3 941 629**
**DE-A- 19 526 224**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 337 222 B1

Printed by Jouve, 75001 PARIS (FR)

## Beschreibung

Die Erfindung betrifft den Einsatz von Polysäuren oder von Polysäuren zusammen mit Salzen von Polysäuren mit einer engen Molmassenverteilung in Dentalmassen.

Polysäuren werden seit etwa Ende der sechziger Jahre in Dentalmassen eingesetzt. Beispielsweise wurde Polyacrylsäure als Grundsubstanz in Zink-Polycarboxylatzementen (D. C. Smith, Br. Dent. J. 125 (1968), 381-384) oder auch in Glasionomerzementen (ASPA I, Wilson und Kent, 1969; DE 20 61 513 A) eingesetzt.

Während Zink-Polycarboxylatzemente bis heute auf Polyacrylsäure basieren, wurden die Glasionomerzemente hinsichtlich der chemischen Zusammensetzung der Polysäure weiterentwickelt.

Aus S. Crisp, B. E. Kent, B. G. Lewis, A. J. Ferner und A. D. Wilson, J. Dent. Res. 59, (1980), 1055-1063 sind beispielsweise Copolymersäuren aus Acrylsäure und Itaconsäure zur Verwendung in Glasionomerzementen bekannt. Außerdem sind aus EP 0 024 056 A1 Glasionomerzemente auf Basis eines Copolymeren aus Acrylsäure und Maleinsäure bekannt.

Der Einsatz von Polyvinylphosphonsäure ist beispielsweise aus den EP 0 340 016 A, EP 0 431 440 A, US 5,179,135, GB 2 272 222 A und US 5,601,640 bekannt.

Die Verwendung von Copolymersäuren aus Acrylsäure und Vinylphosphonsäure ist in der GB 2 291 060 A, der Einsatz von aminosäurefunktionalisierten Polycarbonsäuren in der US 5,369,142 beschrieben.

Üblicherweise werden in Dentalmassen, beispielsweise bei Polyelektrolytzementen und insbesondere bei Zink-Polycarboxylatzementen und Glasionomerzementen, die Polysäuren als hoch konzentrierte, beispielsweise ca. 30 bis 50 %-ige wässrige Lösungen eingesetzt.

Der Gesamtanteil an Polysäure in dentalen Zementen wird häufig einerseits möglichst hoch gewählt, um maximale Festigkeiten des ausgehärteten Zementes zu erreichen, andererseits muss das System beim Mischen und Applizieren eine möglichst geringe Viskosität aufweisen, damit eine Ausbringbarkeit, beispielsweise aus Kapseln gewährleistet ist. Eine Verringerung des Gesamtgehaltes an Polysäure in dentalen Zementen geht allgemein und besonders bei hochfesten Glasionomerzementen mit einer deutlichen Verschlechterung der mechanischen Festigkeit der ausgehärteten Zemente einher.

Es besteht daher ein hoher Bedarf an dentalen Zementen, die bei hohem Gehalt an Polysäure eine verringerte Viskosität besitzen.

Bislang sind verschiedene grundsätzliche Ansatzpunkte zur Lösung dieses' Problems bekannt, die aber mit Nachteilen behaftet sind.

Die Möglichkeiten, die Polysäure in Glasionomerzementen im Pulver unterzubringen, sind insbesondere bei hochfesten Füllungs-Glasionomerzementen beschränkt, da beim Anmischvorgang nur eine sehr kurze Zeit, beispielsweise bis zu wenigen Minuten zum Auflösen der Polysäure im Gesamtsystem zur Verfügung steht.

Grundsätzlich lässt sich die Viskosität eines Polymeren in Lösung dadurch verringern, dass man verzweigte Polymere verwendet. Bei gleichem Molgewicht ist allgemein bei Polymeren die Lösungsviskosität mit zunehmender Verzweigung kleiner. Durch Einsatz von kurzkettenverzweigten, langkettenverzweigten, kammartig verzweigten, sternförmigen, hyperverzeigten oder dendrimeren Polysäuren lassen sich daher niedrig viskosere Zementflüssigkeiten herstellen, die die Gesamtviskosität des Systems günstig beeinflussen.

Der Nachteil solcher Polysäuren besteht einerseits im erheblich höheren Syntheseaufwand und damit den erhöhten Herstellkosten, insbesondere bei der Herstellung definierter Verzweigungsstrukturen, andererseits werden mit zunehmender Verzweigung die Säuregruppen der Polysäure schwerer zugänglich, so dass eine ungewünschte Veränderung der Abbindecharakteristik eintritt. Darüber hinaus werden bei schlechter Zugänglichkeit der Säuregruppen niedrigere Vernetzungsgrade und damit geringere Festigkeiten im Zement erhalten.

In Bull. Kanagawa Dent. Coll (1990), 18(1), 29-32 werden Untersuchungen zum Einfluss der Molmassenverteilung auf die Eigenschaften von Polycarboxylatzementen offenbart. Die untersuchten Polysäuren weisen eine Polydispersität im Bereich von 1,9 bis 5,9 auf. Zusammenfassend wird ausgeführt, dass die Zugfestigkeit von Zementen, die mit einer Poly(acrylsäure) mit einem mittleren Molekulargewicht größer als 100.000 bei gleichzeitig möglichst kleiner Molekulargewichtsverteilung hergestellt wurden, sehr gut sei. Schließlich wird hervorgehoben, dass bei Entwicklungen auf diesem Gebiet sowohl das mittlere Molekulargewicht als auch die Molekulargewichtsverteilung berücksichtigt werden sollten.

Versuche, die von der Anmelderin durchgeführt wurden, um die Reproduzierbarkeit dieser Publikation zu überprüfen, haben ergeben, dass z.B. die Polysäure gemäß Probe L82 eine Molmassenverteilung von 7.4 aufweist und nicht von 1.9, wie angegeben.

Die DE 19 526 224 A befasst sich mit einem Glasionomerzement, welcher ein alpha-beta-ungesättigtes Carbonsäurepolymeres mit einem Gewichtsdurchschnitt-Molekulargewicht im Bereich von 5000 bis 40000 enthält.

Die DE 39 41 629 A betrifft eine Dentalglasionomerzementmasse, die Polymere einer ungesättigten Carbonsäure mit einem Gewichtsmittel des Molekulargewichts von 5000 bis 40000 enthält.

In der DE 24 39 882 werden Polycarboxylat-Zementmassen offenbart, welche als Bestandteil eine Acrylsäure-Copolymerlösung aufweisen. Die Acrylsäure-Copolymerlösung ist gekennzeichnet durch ein mittleres Molekulargewicht von weniger als 20000 und durch eine bestimmte Viskosität. Diese Acrylsäure-Copolymerlösung wird hergestellt durch radikalische Polymerisation der entsprechenden Monomere in wässriger Lösung.

In J. Dent. Res. (1989), 58(2), 89-94 wird der Einfluss des Molekulargewichts von Polysäuren auf die Eigenschaften von GlZen untersucht. Die Polydispersität der untersuchten Polysäuren liegt gemäß den angegebenen Werten im Bereich von 1.58 bis 11.50. Die Werte wurden über Gelpermeationschromatographie erhalten unter Verwendung von Polyethylenoxid-Äquivalten als externen Standard.

Zur reproduzierbaren Bestimmung von Molgewichten und Mogewichtsverteilungen über Gelpermeationschromatographie ist es aber erforderlich, einen Standard zu wählen, der die gleiche chemische Grundstruktur aufweist, wie die zu vermessende Substanz, da diese einen maßgeblichen Einfluss auf die Eluationszeiten hat (J. M. G. Lowie, Chemie und Physik der synthetischen Polymere, Vieweg Verlag 1997, Kapitel 9.14). Insbesondere bei vergleichsweise polaren Polymeren wie Polysäuren, die über Wasserstoffbrückenbindungen starke Assoziatbildungstendenzen zeigen, kann es insbesondere bei Relativmessungen gegen Standards anderer chemischer Strukturen zu starken Verfälschungen der Messergebnisse kommen.

Aufgabe der vorliegenden Erfindung ist es daher, Polysäuren zu finden, die bei Erhalt der Abbinde- und der physikalischen Eigenschaften der abgebundenen Zemente, insbesondere zusammen mit herkömmlichen Zementpulvern, verringerte Lösungsviskositäten der Zementflüssigkeit und damit verbesserte Konsistenzen während des Anmischvorgangs ermöglichen.

Diese Aufgabe wird gelöst durch die Verwendung von Polysäuren oder von Polysäuren zusammen mit Salzen von Polysäuren mit einer Molmassenverteilung von Mw/Mn zwischen 1,0 und 1,7, bevorzugt zwischen 1,0 und 1,5, besonders bevorzugt zwischen 1,0 und 1,3 in Dentalmassen bzw. die Bereitstellung von Dentalmassen, die Polysäuren mit einer derartigen Molmassenverteilung enthalten, wobei die Polysäure über anionische Polymerisation, Gruppentransfer-Polymerisation oder kontrollierte radikalische Polymerisation herstellbar ist.

Unter Mw im Sinne dieser Anmeldung ist das gewichtsmittlere Molekulargewicht, bestimmt mittels wässriger Gelpermeationschromatographie (GPC) zu verstehen, wobei gilt:

$$\mathrm{Mw} = \frac{\sum_i n_i M_i^2}{\sum_i n_i M_i}$$

worin $n_i$ = Zahl der Polymerketten und $M_i$ = Molmasse der Polymerkette bedeuten.

Unter Mn im Sinne dieser Anmeldung ist das zahlenmittlere Molekulargewicht, bestimmt mittels wässriger GPC zu verstehen, wobei gilt:

$$\mathrm{Mn} = \frac{\sum_i n_i M_i}{\sum_i n_i} = \frac{\sum_i n_i M_i}{n}$$

worin n = Gesamtzahl der Polymerketten in einer Probe bedeutet.

Unter Dentalmassen im Rahmen dieser Anmeldung sind insbesondere Zemente, wie Zink-Polycarboxylatzemente, Glasionomerzemente, Resin-Modified-Glasionomerzemente sowie Compomere, soweit sie mit wässrigen Polysäurelösungen formuliert werden können, zu verstehen.

Bei den Dentalmassen handelt es sich um härtbare Massen, d.h. um Massen, die bestimmungsgemäß in einem Zeitraum von 30 sec bis 30 min, vorzugsweise von 2 min bis 10 min von einem viskosen in einen nicht-viskosen Zustand übergehen.

Die Härtungsreaktion kann beispielsweise durch eine Zementreaktion, eine Vernetzungsreaktion und/oder eine Polymerisationsreaktion bewirkt werden.

Ein viskoser, im Unterschied zu einem nicht-viskosen Zustand im obigen Sinne liegt dann vor, wenn die Masse mit dem Zahnarzt geläufigen Applikationsvorrichtungen, wie Spatel, Spritze oder Mischkapsel verarbeitet bzw. appliziert werden kann.

Unter Polysäuren sind Polymere und Copolymere zu verstehen, die mehr als drei Säuregruppen pro Polymermolekül aufweisen. Die Polymere bzw. Copolymere können zusätzlich auch andere funktionelle Gruppen aufweisen. Polysäuren gemäß der vorliegenden Erfindung sind nicht als Einzelsubstanz, sondern als Gemisch verschiedenster

einzelner Moleküle zu verstehen, die jeweils unterschiedliche Molmassen aufweisen.

Unter Säuregruppen sind insbesondere Carbonsäuregruppen, Phosphonsäuregruppen, Phosphorsäuregruppen oder Sulfonsäuregruppen zu verstehen.

Die Begriffe "umfassen" und "enthalten" im Sinne der vorliegenden Erfindung leiten eine nicht-abschließende Aufzählung von Merkmalen ein. Der Begriff "ein" ist äquivalent mit der Angabe von "mindestens ein".

Die erfindungsgemäß eingesetzten Polysäuren weisen eine Molmasse Mw im Bereich von 1.000 bis 500.000, vorzugsweise im Bereich von 2.000 bis 100.000, besonders bevorzugt im Bereich von 5.000 bis 80.000 auf.

Polysäuren, die die erfindungsgemäße Molmassenverteilung aufweisen, besitzen beispielsweise bei einer Konzentration von 38 bis 47 % in Wasser eine Viskosität von 0,49 bis 4,23 Pa.s, bevorzugt bei einer Konzentration von 42 bis 46 % in Wasser eine Viskosität von 1,55 bis 3,11 Pa.s, wobei die Viskosität mit dem Viskosimeter PK100 (Fa. Haake) bei 23°C bestimmt wurde.

Die Tatsache, dass die erfindungsgemäße Molmassenverteilung die beschriebene Aufgabe löst, ist überraschend, weil nach bisherigem Kenntnisstand die hohen Molmassen einer breit verteilten Polysäure für die Zernentfestigkeiten als wesentlich galten (H. J. Posser et al., J. Dent. Res. 1986 und J. Dent. (1977), 5(2), 117-20), während die kleinen Molmassen die Zähflüssigkeit der Polysäurelösung drastisch herabsetzen (H.G. Elias, Makromoleküle Band 1, Hüthing & Wepf Verlag).

Die auf Verzweigungen zurückgehenden Nachteile, wie schlechte Zugänglichkeit der Säuregruppen, werden durch den Einsatz von unverzweigten Polysäuren mit enger Molmassenverteilung besonders einfach vermieden.

Bevorzugt weisen die gemäß der Erfindung verwendbaren Polysäuren Wiederholungseinheiten der Formel (1) auf, die entweder als einzige oder neben weiteren Wiederholungseinheiten auftreten, wobei die Wiederholungseinheiten bei Copolymeren statistisch oder alternierend entlang der Polymerhauptkette angeordnet sein können:

$$-CR^1R^2-CR^3R^4- \qquad (1)$$

C = Kohlenstoff;
$R^1$ = COOH, $PO_3H_2$, $OPO_3H_2$, $SO_3H_2$;
$R^2$ = H, $CH_3$, $C_2H_5$ oder $CH_2COOH$;
$R^3$ = H;
$R^4$ = H, COOH, $COOR^5$, wobei $R^5$ aus folgender Gruppe entnommen ist: linearer, verzweigter oder cyclischer Alkylrest mit 1 bis 12 C-Atomen, bevorzugt $CH_3$, $C_2H_5$, $C_3H_7$, $C_4H_9$; substituierter oder unsubstituierter Arylrest mit 6 bis 18 C-Atomen, bevorzugt Phenyl oder Benzyl; mit 1 bis 5 Heteroatomen aus der Gruppe N, O, S funktionalisierter linearer, verzweigter oder cyclischer Alkylrest mit 1 bis 12 C-Atomen, bevorzugt $CH_3O$, $C_2H_4OR^6$, wobei $R^6$ einen Acylrest darstellt, bevorzugt Acryl oder Methacryl.

Bevorzugte Vertreter von Formel (1) sind:

1. Polyacrylsäure

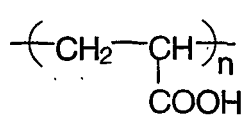

2. Copolymere der Polyacrylsäure mit mehr als 30 Mol-% , bevorzugt mehr als 50 Mol-% Acrylsäureeinheiten;
3. Polymere und Copolymere mit mehr als 30 Mol-%, bevorzugt mehr als 50 Mol-% Einheiten aus der Gruppe: Maleinsäure, Methacrylsäure, Fumarsäure, Itaconsäure, Vinylphosphonsäure, Vinylidendiphosphonsäure, Vinylsulfonsäure oder Phosphorsäurevinylester.

Comonomere hierfür sind bevorzugt entnommen aus der Gruppe: Methacrylsäure, Maleinsäure, Maleinsäureanhydrid, Fumarsäure, Itaconsäure, Itaconsäureanhydrid, Glutaconsäure, Aconitsäure, Citraconsäure, Methaconsäure, Tiglinsäure, Crotonsäure, Muconsäure, Isocrotonsäure, 3-Butensäure, Zimtsäure, Styrolcarbonsäure, Vinylphthalsäure, Abietinsäure, Styrolsulfonsäure, Styrolphosphonsäure, 1-Phenyl-vinylphosphonsäure, Vinylphosphonsäure, Vinylidendiphosphonsäure, Vinylsulfonsäure, Phosphorsäurevinylester, andere säurefunktionelle Monomere sowie deren substituierte Derivate, insbesondere Ester oder Amide bzw. Imide der genannten Säuren mit bis zu 10 C-Atomen im Alkoholat- bzw Amin- oder Iminrest, bevorzugt Acrylsäureester, Methacrylsäureester, Maleinsäureester, oder entsprechende Amide oder Imide, beispielsweise Maleinimid, beispielsweise Methylacrylat, Ethylacrylat, n-Propylacrylat, i-Propylacrylat, n-Butylacrylat, i-Butylacrylat, tert.-Butylacrylat, N-Methylmaleinimid, N-Ethylmaleinimid, Acrylsäureamid, N-Methylacrylsäureamid, N,N-Dimethylacrylsäureamid, N-Ethylacrylsäureamid, 2-Hydroxyethylacrylat, 2-Hydroxyethylmethacrylat, 3-Hydroxypropylacrylat, 3-Hydroxypropylmethacrylat.

Es können zusätzlich oder ausschließlich auch andere Comonomere enthalten sein, beispielsweise Ethylen, Butadien, Isopren.

Die Comonomere können statistisch oder alternierend eingebaut sein, bevorzugt statistisch.

Bevorzugt sind ebenfalls teilverseifte Polyacrylsäureester, insbesondere Poly-(acrylsäure-co-acrylsäuremethylester), Poly-(acrylsäure-co-acrylsäureethylester), Poly-(acrylsäure-co-acrylsäurepropylester), wobei der Propylrest entweder linear oder verzweigt sein kann, Poly-(acrylsäure-co-acrylsäurebutylester, wobei der Butylrest linear oder verzweigt sein kann, Poly-(acrylsäure-co-acrylsäurephenylester), Poly-(acrylsäure-co-acrylsäurebenzylester), jeweils mit Einbauverhältnis der Comonomeren von 1 : 1000 bis 1000 : 1.

Den gemäß dem Stand der Technik verwendeten Polysäuren ist gemeinsam, dass sie mittels gewöhnlicher radikalischer Polymerisation hergestellt werden. Dabei ergeben sich zwangsläufig Materialien, die eine breite Molmassenverteilung mit Mw/Mn sehr viel größer als 1,7, in der Regel ca. 4 bis 6, besitzen.

Polysäuren gemäß der vorliegenden Erfindung, die die erfindungsgemäßen Molmassenverteilungen aufweisen, lassen sich vorzugsweise über anionische Polymerisation, Gruppentransfer-Polymerisation oder kontrollierte radikalische Polymersisation herstellen.

Im folgenden werden geeignete Methoden näher beschrieben:

Eine mögliche Synthese wird über die Ester von Polysäuren durchgeführt, wobei durch lebende anionische oder Gruppentransfer-Polymerisation der entsprechenden Monomeren und anschließende polymeranaloge Verseifung der Estergruppen die Säuren mit der erfindungsgemäßen Molmassenverteilung erhalten werden.

Unter polymeranalogen Reaktionen im Sinne dieser Erfindung sind allgemein Reaktionen am Polymer unter Erhalt des Polymerisationsgrades zu verstehen.

Beispielsweise können. Ester der Polyacrylsäure, wie tertiär-Butylester, n-Butylester, Benzylester, Trimethylsilylester polymerisiert und anschließend die Estergruppen polymeranalog verseift werden, wie es in S. P. Rannard et al., Eur. Polym. J. 29, 407-414 (1993) oder in Y. Morishima et al., Makromol. Chem., Rapid Commun. 2, 507-510 (1981) beschrieben ist.

Ebenso können Polysäuren mit der erfindungsgemäßen Molmassenverteilung durch die Anwendung der kontrollierten radikalischen Polymerisation erhalten werden. Geeignet sind Polymerisationsmethoden, die auf folgenden Systemen beruhen:

- N-Oxylradikal-Initiatorsysteme, wie in A. Goto und T. Fukuda, Makromolekules 1999, 32, 618-623 oder Keoshkerian et al, Macromolecules 1998, 31, 7559-7561 beschrieben;
- Metallkomplex/Halogenid-Systemen, wie in K. Matyjaszewski et al., Macromol. Rapid Commun. 20, 341-346 (1999) beschrieben.

Weitere anwendbare Methoden sind die reverse atom transfer radical polymerisation, wie in G. Moineau et al., Macromolecules 1998, 31, 545-547 beschrieben, sowie die reversible addition-fragmentation chain transfer (RAFT-Methode), wie in Y. K. Chong et al., Macromolecules 1999, 32, 2071-2074 beschrieben.

Die Verseifung von Polysäureestern ist nach herkömmlichen Methoden (Houben Weyl, Methoden der organischen Chemie) möglich, ohne die enge Molmassenverteilung zu verändern. Besonders leicht zu spalten und daher bevorzugt sind beispielsweise Methyl-, Benzyl- oder t-Butylester.

Polysäuren mit der erfindungsgemäßen Molmassenverteilung, im einfachsten Fall Polyacrylsäure, zeigen bei Carboxylatzementen und Glasionomerzementen gleiche Festigkeiten bei nur 25 bis 30% der Lösungsviskosität. Dabei sind die zahlenmittleren Molmassen Mn mit denen herkömmlicher, breit verteilter Polysäuren aus Dentalmassen, beispielsweise Polyacrylsäure vergleichbar.

Mit den beschriebenen Polysäuren lassen sich härtbare Dentalmassen bzw. Zemente herstellen, die beispielsweise über eine Druck-Festigkeit im Bereich von 220 bis 250 MPa (gemessen nach ISO 9917), eine Biegefestigkeit im Bereich von 35 bis 45 MPa (gemessen analog ISO 4049 mit 12 mm langen Prüfkörpern) und/oder eine Oberflächenhärte im Bereich von 400 bis 500 MPa (gemessen gemäß DIN 53456) verfügen.

Brauchbare Pulver/Flüssigkeitsverhältnisse für erfindungsgemäße härtbare Dentalmassen, die durch Mischen eines Pulvers mit einer Flüssigkeit erhalten werden, liegen im Bereich von 0,7 bis 6,0, vorzugsweise im Bereich von 1,0 bis 5,0, besonders bevorzugt im Bereich von 1,5 bis 3,5.

Bei der zahlenmittleren Molmasse Mn werden die unterschiedlich langen Polymerketten entsprechend ihrer Anzahl bei der Mittelwertbildung gewichtet (Lehrbücher der Polymerchemie, beispielsweise H. G. Elias, Makromoleküle Band1, Hüthing & Wepf-Verlag).

Die Kationen der Salze der Polysäuren, die gemäß der Erfindung eingesetzt werden können, sind entnommen aus der Gruppe: Alkali- und Erdalkalielemente, Zink, Aluminium, Scandium, Yttrium, Lanthan. Bevorzugt sind hierbei Na-, Ca- und Al-Salze.

Die im Rahmen dieser Erfindung beschriebenen Polysäuren werden in Dentalmassen eingesetzt, um im Vergleich mit Dentalmassen aus dem Stand der Technik, bei denen Polysäuren mit einem hohen Wert der Molmassenverteilung eingesetzt werden, eine Erniedrigung der Viskosität unter Beibehaltung der physikalischen Parameter der ausgehärteten Dentalmasse zu ermöglichen. Im besonderen werden die Druck- und Biegefestigkeit sowie die Oberflächenhärte nicht erniedrigt, in vielen Fällen sogar verbessert, und die Viskosität der gemischten Dentalmasse vor Beginn der Aushärtung erniedrigt.

Die Polysäuren bzw. Salze von Polysäuren, sofern sie ausreichend löslich sind, können je nach Anwendungsgebiet, als Flüssigkeit oder als Feststoff, beispielsweise erhalten durch Gefrier- oder Sprühtrocknung, eingesetzt werden.

Bevorzugte Dentalmassen im Rahmen dieser Erfindung sind entweder einkomponentig formuliert, wie Compomere, oder zwei- oder mehrkomponentig formuliert, wie Carboxylatzemente, Glasionomerzemente und Resin-Modified-Glasionomerzemente, bei denen die flüssigen von den festen Komponenten getrennt gelagert und direkt vor der Applikation gemischt werden.

Besonders bevorzugt sind Zemente, die im Falle von Glasionomerzementen beispielsweise folgende Bestandteile umfassen können:

(A) 1 bis 60, bevorzugt 5 bis 40 Gew.-%, besonders bevorzugt 10 bis 30 Gew.-% Polysäuren oder Polysäuren zusammen mit Salzen von Polysäuren mit der erfindungsgemäßen Molmassenverteilung;

(B) 35 bis 80, bevorzugt 50 bis 70 Gew.-% Füllstoffe;

(C) 0 bis 20, bevorzugt 1 bis 10 Gew.-% Zusatz- und Hilfsstoffe;

(D) 5 bis 40, bevorzugt 9 bis 30 Gew.-% Wasser,

wobei die Polysäure über anionische Polymerisation, Gruppentransfer-Polymerisation oder kontrollierte radikalische Polymerisation herstellbar ist.

Unter Füllstoffen der Komponente (B) sind prinzipiell reaktive oder nicht reaktive Feststoffe zu verstehen,

Geeignet sind beispielsweise reaktive Fluoraluminiumsilkatgläser aus DE 20 61 513 A1, DE 20 65 824 A1 oder reaktive Gläser, die an der Oberfläche im Vergleich zur durchschnittlichen Zusammensetzung an Calciumionen verarmt sind, wie in DE 29 29 121 A1 beschrieben.

Letzt genannte Gläser sind besonders bevorzugt und können folgende Zusammensetzung aufweisen:

| Bestandteil | Berechnet als | Gew.-% |
|---|---|---|
| Si | $SiO_2$ | 20 bis 60 |
| Al | $Al_2O_3$ | 10 bis 50 |
| Ca | CaO | 1 bis 40 |
| F | F | 1 bis 40 |
| Na | $Na_20$ | 0 bis 10 |
| P | $P_20_5$ | 0 bis 10 |

und insgesamt 0 bis 20 Gew.-%, berechnet als Oxide, an B, Bi, Zn, Mg, Sn, Ti, Zr, La oder anderen dreiwertigen Lanthanoiden, K, W, Ge, sowie weiteren Zusätzen, welche die. Eigenschaften nicht beeinträchtigen und physiologisch unbedenklich sind.

Zusätzlich zu den vorher beschriebenen reaktiven Gläsern können inerte Füllstoffe, wie Quarze, eingesetzt werden.

Unter Komponente (C) sind beispielsweise Zusätze zur Beschleunigung und Verbesserung der Aushärtung, wie sie aus DE 2 319 715 A1 bekannt sind, zu verstehen. Vorzugsweise werden chelatbildende Mittel in Form niedermolekularer Säuremoleküle, wie Weinsäure, zugesetzt.

Ebenso sind darunter Farbpigmente und andere im GIZ-Bereich übliche Hilfsstoffe, beispielsweise zur Verbesserung der Mischbarkeit, zu verstehen.

Neben der Verwendung in konventionellen Glasionomerzementen eignen sich die Polysäuren mit der erfindungsgemäßen Molmassenverteilung auch für den Einsatz in Carboxylatzementen.

Hierbei umfassen die Zusammensetzungen beispielsweise folgende Bestandteile:

(A) 1 bis 60, bevorzugt 5 bis 40 Gew.-%, besonders bevorzugt 10 bis 30 Gew.-% Polysäuren oder Polysäuren

zusammen mit Salzen von Polysäuren mit der erfindungsgemäßen Molmassenverteilung;

(C) 0 bis 20, bevorzugt 1 bis 10 Gew.-% Zusatz- und Hilfsstoffe;

(D) 10 bis 40, bevorzugt 15 bis 30 Gew.-% Wasser;

(E) 30 bis 80, bevorzugt 44 bis 70 Gew.-% Zinkoxid,

wobei die Polysäure über anionische Polymerisation, Gruppentransfer-Polymerisation oder kontrollierte radikalische Polymerisation herstellbar ist.

Compomere oder Resin-Modified-GlZe, die die Polysäuren mit der erfindungsgemäßen Molmassenverteilung enthalten, umfassen beispielsweise folgende Komponenten:

(A) 1 bis 75, bevorzugt 2 bis 69,9 Gew.-%, besonders bevorzugt 10 bis 30 Gew.-% Polysäuren oder Polysäuren zusammen mit Salzen von 0. Polysäuren mit der erfindungsgemäßen Molmassenverteilung;

(D) 5 bis 40 Gew.-%, bevorzugt 10 bis 30 Gew.-% Wasser;

(F) 8,9 bis 70, bevorzugt 10 bis 60 Gew.-%. eines oder mehrerer radikalisch polymerisierbarer Monomere;

(G) 10 bis 85, bevorzugt 15 bis 85 Gew.-% Füllstoffe;

(H) 0,1 bis 5, bevorzugt 0,5 bis 3 Gew.-% Initiatoren und gegebenenfalls Aktivatoren;

(I) 0 bis 30, bevorzugt 0,1 bis 20 Gew.-% Additive, gegebenenfalls Pigmente, Thixotropiehilfsmittel, Weichmacher,

wobei die Polysäure über anionische Polymerisation, Gruppentransfer-Polymerisation oder kontrollierte radikalische Polymerisation herstellbar ist.

Als Komponente (F) werden mono-, di-, oder höherfunktionelle ethylenisch ungesättigte Verbindungen, bevorzugt auf Acrylat- und/oder Methacrylatbasis eingesetzt. Diese können sowohl monomere als auch höhermolekulare oligomere oder polymere Acrylate beinhalten. Ferner können sie in alleiniger Form oder in Abmischung in den Formulierungen eingesetzt werden.

Geeignete Monomere sind beispielsweise die Acrylsäure- und Methacrylsäureester mono-, di- oder höherfunktioneller Alkohole. Exemplarisch seien genannt: 2-Hydroxyethyl(meth)acrylat, Methyl(meth)acrylat, iso-Butyl(meth)acrylat, Ethylenglykoldi(meth)acrylat, Triethylenglykoldi(meth)acrylat (TEGDMA), Hexan-dioldi(meth)acrylat, Dodecandioldi (meth)acrylat und Trimethylolpropantri(meth)-acrylat.

Vorteilhaft einsetzbar sind weiterhin Bisphenol-A-di(meth)acrylat sowie die davon abgeleiteten ethoxy- bzw. propoxylierten Di(meth)acrylate. Auch die in der US 3 066 112 A beschriebenen Monomere auf Basis von Bisphenol-A- und Glycidyl-(meth)acrylat oder deren durch Addition von Isocyanaten entstandenen Derivate sind geeignet.

Besonders geeignet sind auch die in der DE 28 16 823 C1 genannten Diacryl- und Dimethacrylsäureester des Bis(hydroxymethyl)-tricyclo[5.2.1.$0^{2,6}$]-decans und die Diacryl- und Dimethacrylsäureester der mit 1 bis 3 Ethylenoxid- und/oder Propylenoxideinheiten verlängerten Verbindungen des Bis(hydroxymethyl)-tricyclo-[5.2.1.$0^{2,6}$]-decans.

Auch Urethan(meth)acrylate, wie 7,7,9-Trimethyl-4,13-dioxo-5,12-diazahexadecan-1,16-dioxy-dimethacrylat (UDMA), können Bestandteil dieser Komponente sein.

Füllstoffe gemäß Komponente (G) können anorganische Füllstoffe, beispielsweise Quarz, gemahlene Gläser, nicht wasserlösliche Fluoride, wie $CaF_2$, Kieselgele sowie Kieselsäure, insbesondere pyrogene Kieselsäure und deren Granulate sein. Auch Christobalit, Calciumsilikat, Zirkoniumsilikat, Zeolithe, einschließlich der Molekularsiebe, Metalloxidpulver, wie Aluminium- oder Zinkoxide bzw. deren Mischoxide, Bariumsulfat, Yttriumfluorid, Calciumcarbonat sind als Füllstoffe einsetzbar.

Auch fluoridauslösende Füllstoffe, beispielsweise komplexe anorganische Fluoride der allgemeinen Formel $A_nMF_m$ wie in der DE 44 45 266 A1 beschrieben, können eingesetzt bzw. zugesetzt werden. Dabei bedeutet A ein ein- oder mehrwertiges Kation, M ein Metall der III, IV, V Haupt- oder Nebengruppe; n eine ganze Zahl von 1 bis 3 und m eine ganze Zahl von 4 bis 6.

Bestandteil dieser Komponente können auch organische Füllstoffe sein.

Exemplarisch genannt seien übliche perlförmige Polymere und Copolymere auf Basis von Methylmethacrylat, die beispielsweise bei der Fa. Röhm unter der Bezeichnung "Plexidon" oder "Plex" erhältlich sind.

Zum besseren Einbau in die Polymermatrix kann es von Vorteil sein, die genannten Füllstoffe sowie gegebenenfalls röntgenopake Zusatzstoffe mittels eines Silans zu hydrophobieren. Die. Menge des eingesetzten Silans beträgt

üblicherweise 0,5 bis 10 Gew.-%, bezogen auf anorganische Füllstoffe, bevorzugt 1 bis 6 Gew.-%, ganz besonders bevorzugt 2 bis 5 Gew.-%, bezogen auf anorganische Füllstoffe. Übliche Hydrophobierungsmittel sind Silane, beispielsweise Trimethoxymethacryloxypropylsilan.

Die maximale mittlere Korngröße der vorzugsweise anorganischen Füllstoffe beträgt üblicherweise 15 µm, insbesondere 8 µm. Ganz besonders bevorzugt werden Füllstoffe mit einer mittleren Korngröße von < 3 µm eingesetzt.

Unter Initiatoren gemäß Komponente (H) sind Initiatorsysteme, die die radikalische Polymerisation der Monomeren bewirken, beispielsweise Photoinitiatoren und/oder sogenannte Redoxinitiatorsysteme und/oder thermische Initiatoren zu verstehen.

Als Photoinitiatoren eignen sich beispielsweise α-Diketone, wie Campherchinon, in Verbindung mit sekundären und tertiären Aminen, oder Mono- und Bisacylphosphinoxide, wie 2,4,6-Trimethylbenzoyldiphenyl-phosphinoxid und Bis-(2,6-dichlorbenzoyl)-4-n-propylphenyl-phosphinoxid. Es eignen sich aber auch andere Verbindungen dieses Typs, wie sie in EP 0 073 413 A1, EP 0 007 508 A1, EP 0 047 902 A1, EP 0 057 474 A1 und EP 0 184 095 A1 beschrieben sind.

Als Redoxinitiatorsysteme eignen sich organische Peroxidverbindungen zusammen mit sogenannten Aktivatoren. Als organische Peroxidverbindungen kommen dabei insbesondere Verbindungen wie Lauroylperoxid, Benzoylperoxid sowie p-Chlorbenzoylperoxid und p-Methylbenzoylperoxid in Betracht.

Als Aktivatoren eignen sich beispielsweise tertiäre aromatische Amine, wie die aus der US 3 541 068 bekannten N,N-Bis-(hydroxyalkyl)-3,5-xylidine sowie die aus der DE 26 58 530 A1 bekannten N,N-Bis-(hydroxyalkyl)-3,5-di-t-butylaniline, insbesondere N,N-Bis-(β-oxybutyl)-3,5-di-t-butylanilin sowie N,N-Bis-(hydroxyalkyl)-3,4,5-trimethylaniline.

Gut geeignete Aktivatoren sind auch die in der DE 14 95 520 A1 beschriebenen Barbitursäuren und Barbitursäurederivate sowie die in der EP 0 059 451 A1 beschriebenen Malonylsulfamide. Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Dioctyl-4-isobutylmalonylsulfamid.

Zur weiteren Beschleunigung wird die Polymerisation hierbei vorzugsweise in Gegenwart von Schwermetallverbindungen und ionogenem Halogen oder Pseudohalogen durchgeführt. Als Schwermetall ist Kupfer, als Halogenid das Chloridion besonders geeignet. Das Schwermetall wird geeigneterweise in Form löslicher organischer Verbindungen eingesetzt. Ebenso werden die Halogenid- und Pseudohalogenidionen geeigneterweise in Form von löslichen Salzen eingesetzt, beispielsweise genannt seien die löslichen Aminhydrochloride sowie quartäre Ammoniumchloridverbindungen.

Wenn die erfindungsgemäßen Dentalmassen ein Redoxinitiatorsystem aus organischem Peroxid und Aktivator enthalten, so sind vorzugsweise Peroxid und Aktivator in räumlich voneinander getrennten Teilen der erfindungsgemäßen Dentalmasse vorhanden, die erst unmittelbar vor der Anwendung homogen miteinander vermischt werden. Liegen' nebeneinander organisches Peroxid, Kupferverbindung, Halogenid und Malonylsulfamid und/oder Barbitursäure vor, so ist es insbesondere sinnvoll, dass organisches Peroxid, Malonylsulfamid und/oder Barbitursäure und die Kombination Kupferverbindung/Halogenid in drei räumlich voneinander getrennten Bestandteilen vorliegen. Beispielsweise können die Kombination Kupferverbindung/Halogenid, polymerisierbare Monomere sowie Füllstoffe zu einer Paste verknetet sein und die anderen Komponenten in oben beschriebener Weise jeweils mit einer geringen Menge an Füllstoffen oder insbesondere Thixotropie-Hilfsmitteln, wie silanisierter Kieselsäure, und einem Weichmacher, beispielsweise Phthalat, zu zwei separaten Pasten verknetet sein. Andererseits können die polymerisierbaren Monomeren auch zusammen mit organischem Peroxid und Füllern vorliegen. Alternativ ist auch eine Aufteilung von organischem Peroxid, Kupferverbindung, Halogenid und Malonylsulfamid und/oder Barbitursäure gemäß DE 199 28 238 A1 realisierbar.

Als Vertreter der Komponente (I) können beispielsweise zur Erhöhung der Flexibilität der Massen lösliche organische Polymere eingesetzt werden. Geeignet sind beispielsweise Polyvinylacetat sowie die Copolymeren auf Basis Vinylchlorid/Vinylacetat, Vinylchlorid/Vinylisobutylether und Vinylacetat/Maleinsäuredibutylether. Gut geeignet als zusätzliche Weichmacher sind beispielsweise Dibutyl-, Dioctyl- und Dinonylphthalate oder -adipate sowie höhermolekulare Polyphthalsäure- und Adipinsäureester. Als Thixotropiehilfsmittel können neben pyrogenen Kieselsäuren auch modifizierte Schichtsilikate (Bentonite) oder organische Modifikatoren, beispielsweise auf Basis hydrierter Rizinusöle eingesetzt werden. Als Additive können weiterhin Verzögerer, wie sie in der EP 0 374 824 A1 als Komponente (d) beschrieben sind, in den Formulierungen enthalten sein.

Weiterhin sind Behältnisse, die Dentalmassen, enthaltend Polysäuren oder Polysäuren zusammen mit Salzen von Polysäuren mit erfindungsgemäßer Molmassenverteilung, umfassen, beispielsweise Kapseln, Blisterverpackungen, Applikationsspritzen oder Kanülen, Gegenstand dieser Erfindung.

Die Erfindung wird nachfolgend anhand von Beispielen erläutert, ohne dass sie durch diese beschränkt werden soll.

Die Bestimmung der Molmassenverteilung erfolgte hierbei über wässrige Gelpermationschromatographie (GPC) bei pH = 7 gegenüber Polyacrylsäure-Na-Standard mit dem Brechungsindexdetektor RID6a (Fa. Shimadzu). Zur Eichung wurden Polyacrylsäure-Na-Standards (Fa. PSS) eingesetzt. Die Messwerte wurden mit dem Faktor 0,766 auf freie Polyacrylsäure umgerechnet. Die Messung erfolgte bei 23°C.

Die Proben wurden dabei als 0,05%-ige wässrige Lösungen im Laufmittel, einer 0,9 Gew.-%-igen wässrigen

Natriumnitratlösung, der noch 200 ppm Natriumazid zugesetzt sind, vermessen. Die Lösungen wurden durch Zugabe von Natriumhydroxid auf pH = 7 eingestellt.

Für die Messung von maximalen Molekulargewichten bis zu 670.000 g/mol wurde eine Säulenkombination aus Hema3000, HemaBio1000 und HemaBio40 (Fa. PSS) verwendet. Bei maximalen Molekulargewichten bis 1.100.000 g/mol wurde eine Säulenkombination aus Suprema1000, Hema3000 und HemaBio1000 (Fa. PSS) eingesetzt.

Synthesebeispiel 1

Herstellung einer Polyacrylsäure (a)

In einem Reaktionskolben wurde unter Argonatmosphäre ein Gemisch aus 0,65 g 2-Brom-propionsäuremethylester, 0,28 g Kupfer-I-bromid, 0,02 g Kupfer-II-bromid, 0,35 g N,N,N',N",N"-Pentamethyl-diethylentriamin (PMDETA) und 100 g Tertbutylacrylat in 100 ml Dimethylformamid gelöst und auf 70 °C erhitzt. Der Polymerisationsumsatz wurde mittels 1 H-NMR-Spektroskopie überwacht. Nach 24 Stunden war der Ansatz nahezu vollständig polymerisiert.

Nach Lösen der Mischung in 500 ml Toluol wurde mit verdünnter Salzsäure und mit Wasser gewaschen und die organische Phase anschließend eingeengt.

Zum Spalten der Estergruppen wurde das erhaltene Polymer in 500 ml Dioxan gelöst und mit einem Überschuss an konzentrierter Salzsäure fünf Stunden auf 100 °C erhitzt. Durch mehrmaliges Extrahieren mit Diethylether, Einengen am Rotationsverdampfer, zuletzt unter Zugabe von Wasser, wurden Dioxan und Salzsäure entfernt. Die Polyacrylsäure wurde als farblose, wässrige Lösung erhalten, die auf die in Tabelle 1 angegebenen Konzentrationen eingestellt wurde (gemäß Titration mit Lithiumhydroxid in Gegenwart von 1 % Lithiumchlorid).

Synthesebeispiel 2

Herstellung einer Polyacrylsäure (b)

Unter Argonatmosphäre wurde in einem Reaktionskolben ein Gemisch aus 1,22 g 2-Brom-propionsäuremethylester, 0.102 g Kupfer-I-bromid, 0,250 g PMDETA und 87 g Methylacrylat auf 80 °C erhitzt. Nach 15 Stunden war der Ansatz nahezu vollständig durchpolymerisiert. Der Ansatz wurde in 500 ml Toluol gelöst und mit verdünnter Salzsäure sowie mit Wasser extrahiert. Die organische Phase wurde eingeengt.

Das Produkt wurde in 500 ml Dioxan gelöst und mit 100 ml 4 n Kalilauge versetzt. Nach fünf Stunden Erhitzen auf 80 °C war die Esterverseifung vollständig. Das klumpige Gemisch wurde mit Wasser verdünnt und mit saurem Ionenaustauscher behandelt. Mehrmaliges Extrahieren mit Diethylether und Einengen im Vakuum unter Zugabe von Wasser ergab eine gelblich gefärbte wässrige Lösung der Polyacrylsäure.

Die Bestimmung der Molmassenverteilung erfolgte über wässrige GPC bei pH = 7 gegenüber Polyacrylsäure-Na-Standard.

Synthesebeispiel 3

Herstellung von Poly-(acrylsäure-co-butylacrylat) (c)

In einem Reaktionskolben wurden unter Argonatmosphäre 89,8 g n-Butylacrylat, 0,288 g 2,2,6,6-Tetramethyl-1-($\alpha$-phenethoxy)-piperidin (TEMPEP), 0,64 g $\alpha$-D-(+)-Glukose und 0,64 g Natriumhydrogenkarbonat zusammengegeben und fünf Stunden auf 145 °C erhitzt. Dabei wurde ein Polymerisationsumsatz von 40 % errreicht. Der Ansatz wurde im Vakuum eingeengt und mehrfach mit Methanol extrahiert. Man erhielt das Polymer als farbloses Öl.

Zur Teilverseifung wurden 16,4 g des Polybutylacrylates in 160 ml Ethanol gelöst und mit 10 ml Wasser sowie 10,4 g Natriumhydroxid versetzt. Der Ansatz wurde 24 Stunden bei 50 °C gerührt, wobei mehrere Portionen Wasser zugefügt wurden, um ein Ausfallen des Polymers zu verhindern. Der Reaktionsumsatz lag bei 96 %.

Die Reaktionslösung wurde mit Wasser verdünnt und mit saurem Ionenaustauscher behandelt. Anschließend wurde mit Diethylether extrahiert und die wässrige Phase am Rotationsverdampfer eingeengt. Man erhielt eine leicht gelbliche, wässrige Lösung der Poly-(acrylsäure-co-butylacrylat) mit einem Einbauverhältnis von 96 : 4 zugunsten von Acrylsäure (1 H-NMR).

Die Bestimmung der Molmassenverteilung erfolgte über wässrige GPC bei pH = 7.

Synthesebeispiel 4

Herstellung des Natriumsalzes von Polyacrylsäure (a)

80 g einer 40%-igen wässrigen Lösung der Polyacrylsäure gemäß Synthesebeispiel 1 werden mit einer Lösung von 17,8 g (1 Äquivalent) Natriumhydroxid in 50 ml Wasser unter Rühren und Kühlen versetzt. Der Ansatz wird bei Raumtemperatur 3 Stunden nachgerührt und anschließend am Rotationsverdampfer zur Trockene eingeengt. Das Salz des Polymeren wird im Mörser zerkleinert und bei 110°C im Trockenschrank bis zur Gewichtskonstanz getrocknet.

Synthesebeispiel 5

Herstellung des Natriumsalzes von Polyacrylsäure (e)

Die Synthese erfolgte analog Synthesebeispiel 2.
Die Bestimmung der Molmassenverteilung erfolgte über wässrige GPC bei pH = 7 gegenüber Polyacrylsäure-Na-Standard.

Anwendungsbeispiele

Die Polysäuren gemäß den Herstellungsbeispielen wurden in der Anwendung für dentale Carboxylatzemente und Glasionomerzemente geprüft.
Zur Bestimmung von Druckfestigkeiten der abgebundenen Zemente wurde nach der ISO-Norm 9917 verfahren. Biegefestigkeiten wurden in Anlehnung an die ISO-Norm 4049 bestimmt, wobei aber 12 mm lange Probekörper verwendet wurden. Sowohl bei der Messung von Druck- als auch Biegefestigkeiten wurden jeweils Serien von 5 Prüfkörpern vermessen, wobei der relative Fehler bei ca. +-10% pro Messwert lag. Oberflächenhärten wurden nach DIN 53456 bestimmt.
Die Viskositäten der wässrigen Polysäurelösungen wurden mit dem Viskosimeter PK 100 der Firma Haake bei 23°C gemessen.
Konzentrationen wurden durch Titration mit Lithiumhydroxid in Gegenwart von 1 Gew.-% Lithiumchlorid bestimmt.
Die angegebenen Polysäuren wurden entweder mit dem Pulveranteil des Carboxylatzementes Durelon® (Fa. ESPE Dental AG, Seefeld, Deutschland), umfassend mehr als 90 Gew.% Zinkoxid, oder einem Standardglasionomerpulver auf Basis eines Calcium-Strontium-Aluminium-Fluorsilikatglases (GIZ-Glas) (Diamond-Carve-Pulver, Fa. Kemdent, England) angespatelt. Als Vergleichsbeispiel diente jeweils die Flüssigkeit des bereits oben angeführten Produktes Durelon®, eine Polysäure mit einer Molmassenverteilung Mw/Mn = 5,0. Die Ergebnisse sind in Tabelle 2 zusammengefasst.

Tabelle 1: Eigenschaften der Polysäuren mit erfindungsgemäßer-Molmassenverteilung und des Vergleichsbeispiels

| **Polysäure** | **Konzentration [%]** | **Viskosität [Pa.s]** | **Mn** | **Mw/Mn** |
|---|---|---|---|---|
| Vergleich | 42 | 8,13 | 12.000 | 5,0 |
| (a) | 40<br>42<br>45<br>47 | 0,57<br>1,55<br>2,68<br>3,79 | 13.000 | 1,1 |
| (b) | 38<br>42<br>44<br>47 | 0,49<br>1,72<br>2,58<br>4,23 | 12.500 | 1,5 |
| (c) | 46 | 3,11 | 16.000 | 1,3 |
| (d) | 41 | 1,98 | 15.100 | 1,4 |
| (e) | 38 | 2,73 | 60.000 | 1,2 |

Die Viskosität der Polysäuren, die die erfindungsgemäßen Molmassenverteilungen aufweisen, ist bei vergleichbarer Konzentration und vergleichbarem zahlenmittleren Molgewicht Mn niedriger, als die des Vergleichsbeispiels.

Tabelle 2: Festigkeitswerte der Anwendungsbeispiele

| Polysäure | Konzentration [%] | Pulverkomponente | Pulver-Flüssigkeits-Verhältnis | Druckfestigkeit [MPa] | Biegefestigkeit [MPa] |
|---|---|---|---|---|---|
| (a) | 42 | Durelon | 1,5 | 70 | |
| (a) | 42 | Durelon | 2,5 | 97 | |
| Vergleich | 42 | Durelon | 1,5 | 75 | |
| Vergleich | 42 | Durelon | 2,5 | 95 | |
| | | | | | |
| (a) | 42 | GIZ-Glas | 3,5 | 240 | 43 |
| Vergleich | 42 | GIZ-Glas | 3,5 | 237 | 42 |
| | | | | | |
| (b) | 42 | Durelon | 1,5 | 72 | |
| (b) | 42 | Durelon | 2,5 | 96 | |
| (b) | 42 | GIZ-Glas | 3,5 | 243 | 41 |
| Vergleich | 42 | Durelon | 1,5 | 75 | |
| Vergleich | 42 | Durelon | 2,5 | 95 | |
| Vergleich | 42 | GIZ-Glas | 3,5 | 237 | 42 |
| (e) | 38 | GIZ-Glas | 3,5 | 232 | 42 |
| (c) | 46 | Durelon | 1,5 | 74 | |
| (c) | 46 | GIZ-Glas | 3,5 | 246 | 44 |
| Vergleich | 46 | Durelon | 1,5 | (*) | |
| Vergleich | 46 | GIZ-Glas | 3,5 | | |

(*) Die Viskosität der Vergleichspolysäure ist zu hoch, um geeignete Prüfkörper herzustellen.

Die Festigkeitswerte der Dentalmassen, die Polysäuren mit erfindungsgemäßer Molmassenverteilung umfassen, liegen im gleichen Bereich, wie die Werte der Vergleichsmassen.

Die Oberflächenhärten der erfindungsgemäßen Dentalmassen liegen bei Verwendung von GIZ-Glas im Bereich von 400 bis 500 MPa und damit auf gleichem Niveau wie bei Verwendung der Vergleichspolysäure.

Zusammenfassend ermöglichen Polysäuren mit erfindungsgemäßer Molmassenverteilung die Formulierung von Dentalmassen mit niedriger Viskosität bei im Vergleich zu Massen aus dem Stand der Technik nicht-veränderten Festigkeitswerten.

## Patentansprüche

1. Härtbare Dentalmasse, enthaltend mindestens eine Polysäure oder mindestens eine Polysäure mit mindestens einem Salz von mindestens einer Polysäure mit einer Molmassenverteilung Mw/Mn zwischen 1,0 und 1,7, wobei die Polysäure ein Molekulargewicht Mw im Bereich von 1000 bis 500000 aufweist, wobei die Polysäure über anionische Polymerisation, Gruppentransfer-Polymerisation oder kontrollierte radikalische Polymerisation herstellbar ist.

2. Härtbare Dentalmasse gemäß Anspruch 1, wobei die Polysäure linear, unverzweigt und unvernetzt ist.

3. Härtbare Dentalmasse gemäß einem der Ansprüche oder 2, wobei die Polysäure Wiederholungseinheiten der Formel (1) enthält, die entweder als einzige oder neben weiteren Wiederholungseinheiten auftreten, wobei die Wiederholungseinheiten bei Copolymeren statistisch oder alternierend entlang der Polymerhauptkatte angeordnet sein können:

$$-CR^1R^2-CR^3R^4- \qquad (1)$$

C = Kohlenstoff;
$R^1$ = COOH, $PO_3H_2$, $OPO_3H_2$, $SO_3H_2$;
$R^2$ = H, $CH_3$, $C_2H_5$ oder $CH_2COOH$;
$R^3$ = H;
$R^4$ = H; COOH, $COOR^5$, wobei $R^5$ aus folgender Gruppe entnommen ist: linearer, verzweigter oder cyclischer Alkylrest mit 1 bis 12 C-Atomen; substituierter oder unsubstituierter Arylrest mit 6 bis 18 C-Atomen; mit 1 bis 5 Heteroatomen aus der Gruppe N, O, S funktionalisierter linearer, verzweigter oder cyclischer Alkylrest mit 1 bis 12 C-Atomen.

**4.** Härtbare Dentalmasse gemäß einem der Ansprüche 1 bis 3, wobei die Polysäure ausgewählt ist aus der Gruppe: Polyacrylsäure; Poly-(acrylsäurealt-maleinsäure); Poly-(acrylsäure-co-maleinsäure) mit Einbauverhältnis der Comonomeren von 1 : 1000 bis 1000 : 1; Poly-(acrylsäure-co-Itaconsäure) mit Einbauverhältnis der Comonomeren von 1 : 1000 bis 1000 : 1; Poly-(acrylsäure-co-furmarsäure) mit Einbauverhältnis der Comonomeren von 1 : 1000 bis 1000 : 1; teilverseifte Polyacrylsäureester.

**5.** Härtbare Dentalmasse, umfassend:

(A) 1 bis 60 Gew.-% Polysäuren oder Polysäuren und Salze von Polysäuren mit einer Molmassanverteilung Mw/Mn zwischen 1,0 und 1,9;
(B) 35 bis 80 Gew.-% Füllstoffe;
(C) 0 bis 20 Gew.-% Zusatz- und Hilfsstoffe ;
(D) 5 bis 40 Gew.-% Wasser.

wobei die Polysäure über anionische Polymerisation, Gruppentransfer-Polymerisation oder kontrollierte radikalische Polymerisation herstellbar ist.

**6.** Härtbare Dentalmasse, umfassend:

(A) 1 bis 60 Gew.-% Polysäuren oder Polysäuren und Salze von Polysäuren mit einer Molmassenverteilung Mw/Mn zwischen 1,0 und 1,7;
(C) 0 bis 20 Gew.-% Zusatz- und Hilfsstoffe;
(D) 10 bis 40 Gew.-% Wasser,
(E) 30 bis 80 Gew.-% Zinkoxid. wobei die Polysäure über anionische Polymerisation, Gruppentransfer-Polymerisation oder kontrollierte radikalische Polymerisation herstellbar ist.

**7.** Verwendung von Polysäuren oder Polysäuren und mindestens einem Salz von mindestens einer Polysäure mit einer Molmassenverteilung Mw/Mn zwischen 1,0 und 1,7 wobei die Polysäure über anionische Polymerisation, Gruppentransfer-Polymerisation oder kontrollierte radikalische Polymerisation herstellbar ist zur Herstellung von härtbaren Dentalmassen.

**8.** Verwendung nach Anspruch 7 wobei die Verwendung eine Erniedrigung der Viskosität von noch nicht gemischten Komponenten der Dentalmassen oder angemischten Dentalmassen unter Beibehaltung der Druck- und Biegefestigkeit sowie Oberflächenhärte der ausgehärteten Dentalmasse bewirkt.

**9.** Behältnis, umfassend mindestens eine härtbare Dentalmasse, enthaltend mindestens eine Polysäure oder mindestens eine Polysäure und mindestens ein Salz von mindestens einer Polysäure mit einer Molmassenverteilung Mw/Mn zwischen 1,0 und 1,7, wobei die Polysäure über anionische Polymerisation, Gruppentransfer-Polymerisation oder kontrollierte radikalische Polymerisation herstellbar ist.

## Claims

**1.** Curable dental material, comprising at least one polyacid or at least one polyacid with at least one salt of at least one polyacid with a molar mass distribution Mw/Mn between 1.0 and 1.7, in which the polyacid exhibits a molecular weight Mw ranging from 1 000 to 500 000, the polyacid being able to be prepared by anionic polymerization, group transfer polymerization or controlled radical polymerization.

2. Curable dental material according to Claim 1, in which the polyacid is linear, unbranched and noncrosslinked.

3. Curable dental material according to either of Claims 1 and 2, in which the polyacid comprises repeat units of the formula (1) occurring either as sole repeat units or with additional repeat units, the repeat units in copolymers being able to be randomly or alternately arranged along the main polymer chain:

$$-CR^1R^2-CR^3R^4- \qquad (1)$$

C = carbon;
$R^1$ = COOH, $PO_3H_2$, $OPO_3H_2$, $SO_3H_2$;
$R^2$ = H, $CH_3$, $C_2H_5$ or $CH_2COOH$;
$R^3$ = H;
$R^4$ = H, COOH, $COOR^5$, in which $R^5$ is taken from the following group: linear, branched or cyclic alkyl residue with 1 to 12 C atoms; substituted or unsubstituted aryl residue with 6 to 18 C atoms; linear, branched or cyclic alkyl residue with 1 to 12 C atoms functionalized with 1 to 5 heteroatoms from the group consisting of N, O and S.

4. Curable dental material according to one of Claims 1 to 3, in which the polyacid is chosen from the group: polyacrylic acid; poly(acrylic acid-alt-maleic acid); poly(acrylic acid-co-maleic acid) with an incorporation ratio of the comonomers of 1:1 000 to 1 000:1; poly(acrylic acid-co-itaconic acid) with an incorporation ratio of the comonomers of 1:1 000 to 1 000:1; poly(acrylic acid-co-fumaric acid) with an incorporation ratio of the comonomers of 1:1 000 to 1 000:1; partially saponified polyacrylic esters.

5. Curable dental material, including:

(A) 1 to 60% by weight of polyacids or polyacids and salts of polyacids with a molar mass distribution Mw/Mn between 1.0 and 1.9;
(B) 35 to 80% by weight of fillers;
(C) 0 to 20% by weight of additives and auxiliaries;
(D) 5 to 40% by weight of water,

the polyacid being able to be prepared by anionic polymerization, group transfer polymerization or controlled radical polymerization.

6. Curable dental material, including:

(A) 1 to 60% by weight of polyacids or polyacids and salts of polyacids with a molar mass distribution Mw/Mn between 1.0 and 1.7;
(C) 0 to 20% by weight of additives and auxiliaries;
(D) 10 to 40% by weight of water;
(E) 30 to 80% by weight of zinc oxide,

the polyacid being able to be prepared by anionic polymerization, group transfer polymerization or controlled radical polymerization.

7. Use of polyacids or polyacids and at least one salt of at least one polyacid with a molar mass distribution Mw/Mn between 1.0 and 1.7, the polyacid being able to be prepared by anionic polymerization, group transfer polymerization or controlled radical polymerization, in the preparation of curable dental materials.

8. Use according to Claim 7, which brings about a lowering in the viscosity of not yet mixed components of the dental materials or mixed dental materials while retaining the compressive strength, bending strength and surface hardness of the cured dental material.

9. Container, including at least one curable dental material, comprising at least one polyacid or at least one polyacid and at least one salt of at least one polyacid with a molar mass distribution Mw/Mn between 1.0 and 1.7, the polyacid being able to be prepared by anionic polymerization, group transfer polymerization or controlled radical polymerization.

## Revendications

1. Produit dentaire durcissable, contenant au moins un polyacide ou au moins un polyacide avec au moins un sel d'au moins un polyacide ayant une distribution des masses moléculaires Mw/Mn comprise entre 1,0 et 1,7, où le polyacide présente une masse moléculaire Mw comprise dans la plage de 1000 à 500 000, le polyacide pouvant être préparé par polymérisation anionique, polymérisation par transfert de groupes ou polymérisation radicalaire contrôlée.

2. Produit dentaire durcissable selon la revendication 1, dans lequel le polyacide est linéaire, non ramifié et non réticulé.

3. Produit dentaire durcissable selon la revendication 1 ou 2, dans lequel le polyacide contient des motifs répétitifs de formule (1), qui apparaissent à titre individuel ou en plus d'autres motifs répétitifs, les motifs répétitifs pouvant, dans les copolymères, avoir une distribution statistique ou alternée le long de la chaîne principale du polymère :

$$-CR^1R^2-CR^3R^4- \qquad (1)$$

C = carbone ;
$R^1$ = COOH, $PO_3H_2$, $OPO_3H_2$, $SO_3H_2$;
$R^2$ = H, $CH_3$, $C_2H_5$ ou $CH_2COOH$ ;
$R^3$=H;
$R^4$ = H, COOH, $COOR^5$, $R^5$ étant choisi dans le groupe suivant : les résidus alkyle à chaîne droite, ramifiée, ou cycliques, ayant 1 à 12 atomes de carbone ; les résidus aryle substitués ou non substitués ayant 6 à 18 atomes de carbone ; les résidus alkyle à chaîne droite ou ramifiée, ou cycliques, fonctionnalisés par 1 à 5 hétéroatomes du groupe comprenant N, O et S, ayant 1 à 12 atomes de carbone.

4. Produit dentaire durcissable selon l'une des revendications 1 à 3, dans lequel le polyacide est choisi dans le groupe comprenant le poly(acide acrylique) ; le poly(acide acrylique-alt-acide maléique); le poly(acide acrylique-co-acide maléique) avec un rapport entre comonomères de 1:1000 à 1000:1 ; le poly(acide acrylique-co-acide itaconique) avec un rapport entre comonomères de 1:1000 à 1000:1 ; le poly(acide acrylique-co-acide fumarique) avec un rapport entre comonomères de 1:1000 à 1000:1 ; les esters partiellement saponifiés du poly(acide acrylique).

5. Produit dentaire durcissable, comprenant :

   (A) 1 à 60 % en poids de polyacides ou de polyacides et de sels de polyacides ayant une distribution des masses moléculaires Mw/Mn comprise entre 1,0 et 1,9;
   (B) 35 à 80 % en poids de charges ;
   (C) 0 à 20 % en poids d'additifs et d'adjuvants ;
   (D) 5 à 40 % en poids d'eau,

   le polyacide pouvant être préparé par polymérisation anionique, polymérisation par transfert de groupes ou polymérisation radicalaire contrôlée.

6. Produit dentaire durcissable comprenant

   (A) 1 à 60 % en poids de polyacides ou de polyacides et de sels de polyacides ayant une distribution des masses moléculaires Mw/Mn comprise entre 1,0 et 1,7 ;
   (C) 0 à 20 % en poids d'additifs et adjuvants ;
   (D) 10 à 40 % en poids d'eau,
   (E) 30 à 80 % en poids d'oxyde de zinc,

   le polyacide pouvant être préparé par polymérisation anionique, polymérisation par transfert de groupes ou polymérisation radicalaire contrôlée.

7. Utilisation de polyacides ou de polyacides et d'au moins un sel d'au moins un polyacide ayant une distribution des masses moléculaires Mw/Mn comprise entre 1,0 et 1,7, les polymères pouvant être préparés par polymérisation anionique, polymérisation par transfert de groupes ou polymérisation radicalaire contrôlée, pour préparer des produits dentaires durcissables.

8. Utilisation selon la revendication 7, qui provoque une diminution de la viscosité de composants non encore mélangés

des produits dentaires ou des produits dentaires triturés, avec conservation de la résistance à la compression et à la flexion, ainsi que de la dureté superficielle du produit dentaire durci.

**9.** Récipient, comprenant au moins un produit dentaire durcissable, contenant au moins un polyacide ou au moins un polyacide et au moins un sel d'au moins un polyacide ayant une distribution des masses moléculaires Mw/Mn comprise entre 1,0 et 1,7, le polyacide pouvant être préparé par polymérisation anionique, polymérisation par transfert de groupes ou polymérisation radicalaire contrôlée.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2061513 A, Wilson und Kent
- EP 0024056 A1
- EP 0340016 A
- EP 0431440 A
- US 5179135 A
- GB 2272222 A
- US 5601640 A
- GB 2291060 A
- US 5369142 A
- DE 19526224 A
- DE 3941629 A
- DE 2439882
- DE 2061513 A1
- DE 2065824 A1
- DE 2929121 A1
- DE 2319715 A1
- US 3066112 A
- DE 2816823 C1
- DE 4445266 A1
- EP 0073413 A1
- EP 0007508 A1
- EP 0047902 A1
- EP 0057474 A1
- EP 0184095 A1
- US 3541068 A
- DE 2658530 A1
- DE 1495520 A1
- EP 0059451 A1
- DE 19928238 A1
- EP 0374824 A1


### In der Beschreibung aufgeführte Nicht-Patentliteratur


- **D. C. SMITH.** *Br. Dent. J.,* 1968, vol. 125, 381-384
- **S. CRISP ; B. E. KENT ; B. G. LEWIS ; A. J. FERNER ; A. D. WILSON.** *J. Dent. Res.,* 1980, vol. 59, 1055-1063
- *Bull. Kanagawa Dent. Coll,* 1990, vol. 18 (1), 29-32
- *J. Dent. Res.,* 1989, vol. 58 (2), 89-94
- **M. G. LOWIE.** Chemie und Physik der synthetischen Polymere. Vieweg Verlag, 1997
- **H. J. POSSER et al.** *J. Dent. Res.,* 1986
- *J. Dent.,* 1977, vol. 5 (2), 117-20
- **H.G. ELIAS.** Makromoleküle. Hüthing & Wepf Verlag, vol. 1
- **S. P. RANNARD et al.** *Eur. Polym. J.,* 1993, vol. 29, 407-414
- **Y. MORISHIMA et al.** *Makromol. Chem., Rapid Commun.,* 1981, vol. 2, 507-510
- **A. GOTO ; T. FUKUDA.** *Makromolekules,* 1999, vol. 32, 618-623
- **KEOSHKERIAN et al.** *Macromolecules,* 1998, vol. 31, 7559-7561
- **K. MATYJASZEWSKI et al.** *Macromol. Rapid Commun.,* 1999, vol. 20, 341-346
- **G. MOINEAU et al.** *Macromolecules,* 1998, vol. 31, 545-547
- **Y. K. CHONG et al.** *Macromolecules,* 1999, vol. 32, 2071-2074
- **H. G. ELIAS.** Makromoleküle. Hüthing & Wepf-Verlag, vol. 1